(19) Europäisches Patentamt
European Patent Office
Office européen des brevets

(11) Veröffentlichungsnummer: **0 234 449**
**A2**

(12) **EUROPÄISCHE PATENTANMELDUNG**

(21) Anmeldenummer: **87102066.5**

(51) Int. Cl.⁴: **C07D 319/20**

(22) Anmeldetag: **13.02.87**

(30) Priorität: **25.02.86 DE 3605977**

(43) Veröffentlichungstag der Anmeldung:
**02.09.87 Patentblatt 87/36**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Marhold, Albrecht, Dr.**
**Carl-Duisberg-Strasse 329**
**D-5090 Leverkusen 1(DE)**

(54) **Verfahren zur Herstellung von fluorierten o-Diamino-benzo-1,4-dioxenen, das neue 2,2,3,3-Tetrafluor-6,7-diamino-benzo-1,4-dioxen und neue fluorierte 0-Dinitro-benzo-1,4-dioxene.**

(57) Fluorierte o-Amino-benzo-1,4-dioxene werden hergestellt, indem man fluorierte Benzo-1,4-dioxene oder fluorierte Mononitro-benzo-1,4-dioxene mit einem speziellen Nitriersäuregemisch zu fluorierten o-Dinitro-benzo-1,4-dioxenen umsetzt und reduziert. Die Erfindung betrifft weiterhin neue fluorierte o-Dinitro-benzo-1,4-dioxene und das neue 2,2,3,3-Tetrafluor-6,7-diamino-benzo-1,4-dioxen.

EP 0 234 449 A2

Xerox Copy Centre

# Verfahren zur Herstellung von fluorierten o-Diamino-benzo-1,4-dioxenen, das neue 2,2,3,3-Tetrafluor-6,7-diamino-benzo-1,4-dioxen und neue fluorierte o-Dinitro-benzo-1,4-dioxene

Es ist bekannt, daß man 2,2,3-Trifluor-und 2-Chlor-2,3,3-trifluor-2,3-dihydro-1,4-benzodioxin-6,7-diamin-dihydrochlorid herstellen kann, indem man in 2,2,3-Trifluor-oder 2-Chlor-2,3,3-trifluor-2,3-dihydro-1,4-benzodioxin (= 2,2,3-Trifluor-oder 2-Chlor-2,3,3-trifluor-benzo-1,4-dioxen) zunächst unter relativ milden Nitrierbedingungen eine Nitrogruppe in 6-oder 7-Stellung einführt, diese zu einer Aminogruppe reduziert, danach die Aminogruppe in eine Acetaminogruppe umwandelt, dann, unter härteren Nitrierbedingungen (100 %ige $HNO_3$ in Methylenchlorid) erneut eine Nitrogruppe in o-Stellung zur vorhandenen Acetaminogruppe einführt, nun die Acetaminogruppe wieder in eine Aminogruppe rücküberführt und abschließend die Aminonitro-Verbindung zur Diaminoverbindung reduziert (siehe EP-A O 127 763, Beispiele 10 und 11).

Dieses Verfahren ist wegen der vielen Einzelschritte sehr aufwendig und umständlich. Die Herstellung von fluorierten Dinitro-benzo-1,4-dioxenen wird dabei bewußt vermieden, die Herstellung von 2,2,3,3-Tetrafluor-6,7-diamino-benzo-1,4-dioxen nicht in Erwägung gezogen.

Es wurde nun ein Verfahren zur Herstellung von fluorierten o-Amino-benzo-1,4-dioxenen gefunden, das dadurch gekennzeichnet ist, daß man fluorierte Benzo-1,4-dioxene oder fluorierte Mononitro-benzo-1,4-dioxene mit einem Nitriersäuregemisch, hergestellt aus 1 Gewichtsteil mindestens 96 gew.-%iger Salpetersäure und 6 bis 20 Gewichtsteilen (vorhandenes $SO_3$ als Schwefelsäure gerechnet) 10 bis 65 Gew.-% $SO_3$ enthaltender Schwefelsäure, zu fluorierten o-Dinitro-benzo-1,4-dioxenen umsetzt und diese reduziert.

In das erfindungsgemäße Verfahren können beispielsweise fluorierte Benzo-1,4-dioxene der Formel (I) eingesetzt werden

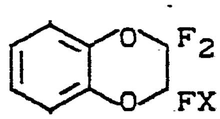

(I),

in der

X für Wasserstoff, Chlor oder Fluor steht,

Mit dem angegebenen Nitriersäuregemisch werden fluorierte Benzo-1,4-dioxene in einer Stufe dinitriert.

Man kann in Verbindungen der Formel (I) auch eine erste Nitrogruppe auf an sich bekannte Weise, z.B. wie in EP-A O 127 763 beschrieben oder analog dazu, in 6-oder 7-Stellung einführen, so fluorierte Mononitro-benzo-1,4-dioxene erhalten, und erst dann das für die Dinitrierung erfindungsgemäß erforderliche Nitriersäuregemisch anwenden.

Verbindungen der Formel (I) können beispielsweise erhalten werden, indem man Brenzkatechin mit Trifluorchlorethylen umsetzt, gegebenenfalls noch chloriert und fluoriert (siehe DE-OS 2 848 531).

Das erfindungsgemäß anzuwendende Nitriersäuregemisch enthält vorzugsweise 1 Gewichtsteil Salpetersäure und 5 bis 20 Gewichtsteile (vorhandenes $SO_3$ als Schwefelsäure gerechnet) 10 bis 35 Gew.-% $SO_3$ enthaltende Schwefelsäure. Das Nitriersäuregemisch kann beispielsweise in einer Menge von 150 bis 400 Gew.-%, bezogen auf fluoriertes Benzo-1,4-dioxen bzw. von 120 bis 300 Gew.-%, bezogen auf fluorierte Mononitro-benzo-1,4-dioxene, eingesetzt werden.

Das Reaktionsgemisch kann gegebenenfalls zusätzlich ein inertes Lösungsmittel enthalten, beispielsweise Dichlormethan, 1,2-Dichlorethan oder Trifluortrichlorethan. In diesen Fällen kann man mit relativ geringen Mengen $SO_3$ enthaltender Schwefelsäure auskommen, beispielsweise mit 6 bis 8 Gew.-Teilen - (vorhandenes $SO_3$ als Schwefelsäure gerechnet) 10 bis 65 Gew.-% $SO_3$ enthaltender Schwefelsäure pro Gewichtsteil mindestens 96 gew.-%iger Salpetersäure. Inerte Lösungsmittel können z.B. in Mengen von 50 bis 500 Gew.-%, bezogen auf fluoriertes Benzo-1,4-dioxen, eingesetzt werden.

Geeignete Reaktionstemperaturen zum Erhalten von fluorierten o-Dinitro-benzo-1,4-dioxenen sind beispielsweise solche im Bereich von 20 bis 100°C. Die Reaktionszeiten können beispielsweise zwischen 20 Minuten und 6 Stunden liegen.

Nach Beendigung der Nitrierreaktion kann man das Reaktionsgemisch beispielsweise so aufarbeiten, daß man es zunächst abkühlt, auf Eis, Eiswasser oder kaltes Wasser austrägt, falls das Nitriersäuregemisch kein derartiges Lösungsmittel enthielt, ein inertes, mit Wasser nicht mischbares Lösungsmittel, z.B. Dichlormethan, hinzumischt, die organische Phase von der wäßrigen Phase trennt, die organische Phase mit Wasser nachwäscht, trocknet (z.B. mit Natriumsulfat) und das Lösungsmittel entfernt, z.B. durch Destillation, gegebenenfalls unter vermindertem Druck.

Man erhält so fluorierte o-Dinitro-benzo-1,4-dioxene, beispielsweise solche der Formel (II)

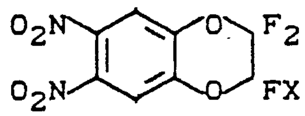

(II),

in der
X für Wasserstoff, Chlor oder Fluor steht,
im allgemeinen in Ausbeuten von über 85 % der Theorie und in Reinheiten von über 97 %.

Die Reduktion der fluorierten o-Dinitro-benzo-1,4-dioxene zu den entsprechenden fluorierten o-Diamino-benzo-1,4-dioxenen, beispielsweise zu solchen der Formel (III)

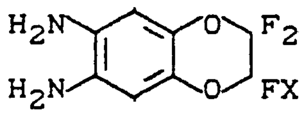

(III),

in der
X für Wasserstoff, Chlor oder Fluor steht,
kann auf verschiedene, an sich bekannte Art und Weise erfolgen, z.B. durch katalytische Hydrierung. Als Katalysatoren kommen dabei beispielsweise solche vom Raney-Typ in Frage, insbesondere Raney-Nickel. Im allgemeinen kann man eine solche katalytische Hydrierung bei Wasserstoffdrucken von 10 bis 100 bar, Temperaturen im Bereich von 20 bis 80°C und in Gegenwart eines Lösungsmittels, z.B. Methanol durchführen.

Nach einer derartigen katalytischen Hydrierung kann man die fluorierten o-Diamino-benzo-1,4-dioxene beispielsweise gewinnen, indem man den Katalysator abfiltriert und das Lösungsmittel durch Destillation, gegebenenfalls bei vermindertem Druck, entfernt. Falls gewünscht kann man die so isolierten fluorierten o-Diamino-benzo-1,4-dioxene durch Umkristallisation, z.B. aus Cyclohexan, oder durch Destillation im Hochvakuum weiter reinigen.

Die erfindungsgemäß erhältlichen fluorierten o-Diamino-benzo-1,4-dioxene können durch Umsetzung mit Kohlensäurederivaten der Formel (IV)

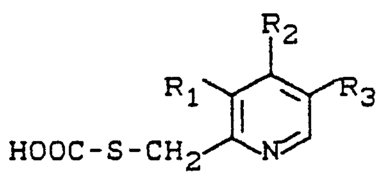

(IV),

in der
$R_1$,$R_2$ und $R_3$ gleich oder verschieden sind und für Wasserstoff oder einen $C_1$-bis $C_3$-Alkylrest stehen und $R_2$ außerdem auch für einen $C_1$-bis $C_3$-Alkoxyrest stehen kann,
in 2-Benzimidazolyl-2-pyridylmethyl-sulfid-oder -sulfoxid-derivate überführt werden, die in der Human-und Veterinärmedizin als ausgezeichnete, die Magensäuresekretion hemmende und den Magen und den Darm - schützende Verbindungen angewendet werden können (vgl. EP-A O 127 763).

Das erfindungsgemäße Verfahren ist viel einfacher als das bisher bekannte Verfahren zur Herstellung von fluorierten o-Diamino-benzo-1,4-dioxenen und gestattet deren Herstellung in guten Ausbeuten. Es ist besonders überraschend, daß dies erzielt wird, obwohl bisher Dinitroverbindungen als Zwischenstufe bewußt vermieden wurden, indem zuerst eine Nitrogruppe eingeführt, diese in eine Acetaminogruppe überführt und erst danach erneut eine Nitrogruppe eingeführt wurde (vgl. EP-A O 127 763). Außerdem ist überraschend, daß es erfindungsgemäß gelingt beide Nitrogruppen mit hoher Selektivität in gegenseitiger ortho-Stellung einzuführen.

Die vorliegende Erfindung betrifft weiterhin neue fluorierte o-Dinitro-benzo-1,4-dioxene der Formel (II)

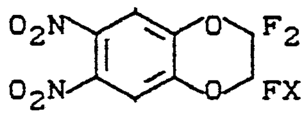

(II),

in der

X für Wasserstoff, Chlor oder Fluor steht.

Die Herstellung und Verwendbarkeit, sowie die technischen Vorurteile gegen die Verbindungen der Formel (II) und die Vorteile, die mit ihnen realisierbar sind, sind bereits weiter oben beschrieben worden.

Die vorliegende Erfindung betrifft weiterhin das neue fluorierte o-Diamino-benzo-1,4-dioxen der Formel - (V)

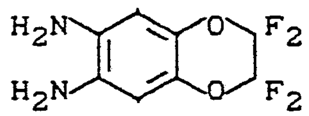

(V).

Diese Verbindung kann auch als 2,2,3,3-Tetrafluor-6,7-diamino-benzo-1,4-dioxen bezeichnet werden.

Diese Verbindung ist bisher nicht beschrieben, insbesondere nicht in der EP-A 0 127 763. Ihre Herstellung und Verwertbarkeit sind bereits weiter oben beschrieben worden.

## Beispiele

### Beispiel 1

75 g 98-gew.-%ige Salpetersäure und 290 ml 25 Gew.-% $SO_3$ enthaltende Schwefelsäure wurden bei 60°C vorgelegt und 1 Mol 2,2,3,3-Tetrafluor-6-nitro-benzo-1,4-dioxen, erhalten gemäß DE-OS 32 23 505 zugetropft. Die Temperatur wurde 2 Stunden bei 60°C belassen, danach wurde abgekühlt und das Reaktionsgemisch auf 1 kg Eis ausgetragen. Dann wurde Dichlormethan zugegeben, die organische von der wäßrigen Phase getrennt, die organische Phase mit Wasser gewaschen mit Natriumsulfat getrocknet und am Rotationsverdampfer vom Lösungsmittel befreit. So wurde in einer Ausbeute von 95 % der Theorie und in einer Reinheit von 98,5 % 2,2,3,3-Tetrafluor-6,7-dinitro-benzo-1,4-dioxen (Formel II, X = F) mit einem Schmelzpunkt von 56 bis 58°C erhalten.

### Beispiel 2

Es wurde verfahren wie in Beispiel 1, jedoch wurde 1 Mol 2-Chlor-2,3,3-trifluor-7-nitrobenzo-1,4-dioxen eingesetzt und in einer Ausbeute von 87,5 % der Theorie und in einer Reinheit von 98 % 2-Chlor-2,3,3-trifluor-6,7-dinitro-benzo-1,4-dioxen (Formel II, X = Cl) mit einem Schmelzpunkt von 54 bis 57°C erhalten.

### Beispiel 3

Es wurde verfahren wie in Beispiel 1, jedoch wurde 1 Mol 2,3,3-Trifluor-7-nitrobenzo-1,4-dioxen eingesetzt und in einer Ausbeute von 87 % der Theorie und in einer Reinheit von 99,7 % 2,3,3-Trifluor-6,7-dinitro-benzo-1,4-dioxen (Formel II, X = H) mit einem Schmelzpunkt von 81 bis 83°C erhalten.

### Beispiele 4

In einem Hydrierautoklaven wurden 100 g der nach Beispiel 1 erhaltenen Dinitroverbindung zusammen mit 500 ml Methanol und 10 g Raney-Nickel vorgelegt und bei 25 bis 45°C und 30 bis 50 bar Wasserstoffdruck bis zur Druckkonstanz hydriert. Danach wurde das Raney-Nickel durch Filtration entfernt, das Methanol bei vermindertem Druck abgezogen und das erhaltene Produkt im Hochvakuum destilliert. Zur

Bestimmung des Schmelzpunktes wurde eine kleine Probe davon aus Cyclohexan umkristallisiert. In einer Ausbeute von 86 % der Theorie wurde 2,2,3,3-Tetrafluor-6,7-diamino-benzo-1,4-dioxen (Formel III, X = F) mit einem Siedepunkt von 110 bis 115°C bei 0,2 mbar und einem Schmelzpunkt von 87 bis 88°C erhalten.

Beispiel 5

Es wurde verfahren wie in Beispiel 4, jedoch wurden 100 g der gemäß Beispiel 2 erhaltenen Dinitroverbindung eingesetzt und in einer Ausbeute von 71 % der Theorie 2-Chlor-2,3,3-trifluor-6,7-diamino-benzo-1,4-dioxen (Formel III, X = Cl) mit einem Siedepunkt von 123 bis 130°C bei 0,1 mbar und einem Schmelzpunkt von 85 bis 87°C erhalten.

Beispiel 6

Es wurde verfahren wie in Beispiel 4, jedoch wurden 100 g der gemäß Beispiel 3 erhaltenen Dinitroverbindung eingesetzt und in einer Ausbeute von 82 % der Theorie 2,3,3-Trifluor-6,7-diamino-benzo-1,4-dioxen (Formel III, X = H) mit einem Siedepunkt von 120 bis 124°C bei 0,1 mbar und einem Schmelzpunkt von 65 bis 67°C erhalten.

Beispiel 7

400 g Mischsäure O (Nitriersäuremischung aus 33 % Salpetersäure und 67 % Schwefelsäure) wurden bei 20°C vorgelegt und 200 g 2-Chlor-2,3,3-trifluor-benzo-1,4-dioxen zugetropft. Es wurde 1 Stunde bei 20°C und 1 Stunde bei 40°C gerührt, dann wieder auf 20°C abgekühlt und 100 ml 28 Gew.-% $SO_3$ enthaltendes Oleum zugetropft. Nach 1 Stunde Reaktionszeit bei 20°C und 1 Stunde bei 50°C wurde erneut abgekühlt und auf Eis ausgetragen. Dann wurde in Dichlormethan aufgenommen, die organische Phase mit Wasser gewaschen und nach dem Trocknen vom Lösungsmittel befreit. Es verblieben 245 g rohe Dinitroverbindung (Schmelzpunkt 45 bis 47°C), die durch Waschen mit kaltem Methanol gereinigt wurde. Die Verbindung (Formel (II), X = Cl) war dann nach dünnschichtchromatographischer Untersuchung einheitlich und hatte einen Schmelzpunkt von 54 bis 55°C.

Beispiel 8

Bei 10°C wurden 80 g 2,3,3-Trifluor-benzo-1,4-dioxen in 100 ml Dichlormethan vorgelegt und 180 g Mischsäure O (Zusammensetzung siehe Beispiel 7) zugetropft. Danach wurde 1 Stunde bei 20°C und 1 Stunde bei 40°C nachgerührt, noch 80 ml 20 Gew.-% $SO_3$ enthaltendes Oleum zugetropft und für eine weitere Stunde bei 40°C gehalten. Nach Abkühlen auf 20°C wurde auf Eis gegossen, die organische Phase abgetrennt, mit Wasser gewaschen, anschließend mit Natriumsulfat getrocknet, filtriert und vom Lösungsmittel befreit. Durch Destillation wurden mit einem Siedepunkt von 137 bis 140°C bei 0,2 mbar 104 g Dinitroverbindung (Formel (II), X = H) erhalten.

**Ansprüche**

1. Verfahren zur Herstellung von fluorierten o-Diamino-benzo-1,4-dioxenen, dadurch gekennzeichnet, daß man fluorierte Benzo-1,4-dioxene oder fluorierte Mononitro-benzo-1,4-dioxene mit einem Nitriersäuregemisch, hergestellt aus 1 Gew.-Teil mindestens 96 gew.-%iger Salpetersäure und 6 bis 20 Gew.-Teilen (vorhandenes $SO_3$ als Schwefelsäure gerechnet) 10 bis 65 Gew.-% $SO_3$ enthaltender Schwefelsäure, zu fluorierten o-Dinitro-benzo-1,4-dioxenen umsetzt und diese reduziert.

2. Verfahren nach Anspruch 1, dadurch gekennzeichnet, daß man fluorierte Benzo-1,4-dioxene der Formel

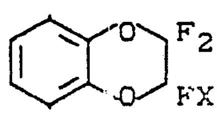

in der
X für Wasserstoff, Chlor oder Fluor steht,
einsetzt.

3. Verfahren nach Anspruch 1, dadurch gekennzeichnet, daß man fluorierte Mononitro-benzo-1,4-dioxene einsetzt.

4. Verfahren nach Ansprüchen 1 bis 3, dadurch gekennzeichnet, daß das Nitriersäuregemisch 1 Gew.-Teil Salpetersäure und 5 bis 20 Gew.-Teile (vorhandenes $SO_3$ als Schwefelsäure gerechnet) 10 bis 35 Gew.-% $SO_3$ enthaltende Schwefelsäure enthält.

5. Verfahren nach Ansprüchen 1 bis 4, dadurch gekennzeichnet, daß das Nitriersäuregemisch in einer Menge von 150 bis 400 Gew.-%, bezogen auf fluoriertes Benzo-1,4-dioxen bzw. von 120 bis 300 Gew.-%, bezogen auf fluoriertes Mononitro-benzo-1,4-dioxen, eingesetzt wird.

6. Verfahren nach Ansprüchen 1 bis 5, dadurch gekennzeichnet, daß das Reaktionsgemisch zusätzlich ein inertes Lösungsmittel enthält.

7. Verfahren nach Ansprüchen 1 bis 6, dadurch gekennzeichnet, daß man die Umsetzung mit dem Nitriersäuregemisch bei 20 bis 100°C durchführt.

8. Verfahren nach Ansprüchen 1 bis 7, dadurch gekennzeichnet, daß man zur Reduktion eine katalytische Hydrierung mit Raney-Nickel bei Wasserstoffdrucken von 10 bis 100 bar, bei Temperaturen im Bereich von 20 bis 80°C und in Gegenwart eines Lösungsmittels durchführt.

9. Neue fluorierte o-Dinitro-benzo-1,4-dioxene der Formel

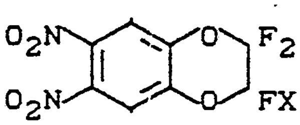

in der
X für Wasserstoff, Chlor oder Fluor steht.

10. Das neue fluorierte o-Diamino-benzo-1,4-dioxen der Formel